# EUROPEAN PATENT SPECIFICATION

(11) **EP 1 597 383 B1**
(45) Date of publication and mention of the grant of the patent: **09.12.2009**
(21) Application number: 04710148.0
(22) Date of filing: 11.02.2004
(51) Int. Cl.: C12Q 1/00, C12Q 1/68, C07K 1/00, C07K 14/00, C07H 21/02

(54) **VARIANTS OF CYTOCHROME C AND METHODS OF DIAGNOSIS OF THROMBOCYTOPENIA**
CYTOCHROM-C-VARIANTEN UND VERFAHREN ZUR DIAGNOSE VON THROMBOZYTOPENIE
VARIANTES DE CYTOCHROME C ET METHODES DE DIAGNOSE DE THROMBOCYTOPENIE

(30) Priority: 11.02.2003 US 446416 P
(43) Date of publication of application: 23.11.2005
(73) Proprietor: Pacific Edge Biotechnology Limited, Dunedin 9001 (NZ)
(72) Inventor: MORISON, Ian M., Belleknowes, Dunedin 9001 (NZ)
(74) Representative: Woods, Geoffrey Corlett
(86) International application number: PCT/US2004/003786
(87) International publication number: WO 2004/072256

(56) References cited:
- BUIJS A ET AL: "A novel CBFA2 single-nucleotide mutation in familial platelet disorder with propensity to develop myeloid malignancies." BLOOD. 1 NOV 2001, vol. 98, no. 9, 1 November 2001 (2001-11-01), pages 2856-2858, XP002397005 ISSN: 0006-4971
- BALDUINI CARLO L ET AL: "Inherited thrombocytopenias: from genes to therapy." HAEMATOLOGICA. AUG 2002, vol. 87, no. 8, August 2002 (2002-08), pages 860-880, XP002397006 ISSN: 0390-6078
- SAVOIA A ET AL: "An autosomal dominant thrombocytopenia gene maps to chromosomal region 10p." AMERICAN JOURNAL OF HUMAN GENETICS. NOV 1999, vol. 65, no. 5, November 1999 (1999-11), pages 1401-1405, XP002397038 ISSN: 0002-9297
- DATABASE GENBANK [Online] 24 April 2002 BIRREN ET AL: 'Homo sapiens chromosome 15, clone RP11-302J23', XP003003441 Database accession no. AC018781
- DATABASE GENBANK [Online] 06 February 2003 BIRREN ET ALL: 'Homo sapiens chromosome 15, clone RP11-79C23', XP003003442 Database accession no. AC138701
- BROWN C. ET AL: 'In Situ Hybridization with Riboprobes: An Overview for Veterinary Pathologist' VET PATHOL vol. 35, no. 3, May 1998, pages 159 - 167, XP008057267

## Description

### Claim of Priority

This application claims priority to U.S. provisional patent application serial No. 60/446,416 filed on February 11, 2003, incorporated herein fully by reference.

### Field of the Invention

This invention relates to novel variants of human cytochrome C ("HCS"). Specifically, this invention relates to mutant forms of cytochrome C, the involvement of mutants of cytochrome C in thrombocytopenia, and diagnosis of thrombocytopenia.

### BACKGROUND OF THE INVENTION

Platelets, also know as thrombocytes, are involved in formation of blood clots and wound healing. Platelets are small circulating fragments of bone marrow cells termed megakaryocytes which break down into small pieces of cells containing portions of the cell's original membrane and importantly, biologically active inclusion granules. Animals that have wounds, either through injury, disease or surgery, can experience severe consequences of bleeding, and in most severe forms, bleeding can lead to exsanguination and death. Thus, it is important for animals to have effective mechanisms that can repair damage to blood vessels and thereby reduce or stop bleeding. Inclusion granules contain numerous substances, including molecules that canparticipate in initiating or maintaining clot forming processes. Thus, when functioning normally, a break in a blood vessel typically results in a series of events that lead to platelet adherence at or near the site of the break. Release of platelet factors typically promote the formation of a fibrin clot at the site of damage, thereby providing a structural barrier to leakage ofblood out of the blood vessel. Thus, an important factor in a body's mechanisms for vascular repair includes normally functioning platelets in sufficient numbers. Platelets have a finite half-life in the blood. Thus, for the number of platelets to remain in a proper range, new platelets must be continuously made to replace those that are lost through the normal limitation of their effective life span, or through use in clot formation. The processes responsible for development of megakaryocytes and their maturation and platelet formation is under extensive study.

Thrombocytopenia is a medical condition characterized by decreased numbers of platelets in the blood. Implications of this condition can vary from a relatively minor form in which blood clotting is somewhat slower than normal, but is sufficiently short to provide adequate clot formation. In more severe forms, thrombocytopenia is a life-threatening condition in whichmajor internal or external bleeding can occur. In patients having severe thrombocytopenia, the usual therapy is through infusion of platelets derived from donors. However, due to hazards associated with transfusion of blood products, patients can become inadvertently infected with infections, including bacterial and viral organisms. With the substantial increases blood-borne transmission of viral diseases, such as human immune deficiency virus (HIV) associated with acquired immune deficiency syndrome (AIDS), hepatitis A, hepatitis C, delta factor and other infectious agents, it is increasingly important to prevent transmission of such diseases to people in need of platelets.

Unfortunately, there are few therapies available for treating transfusion-related viral infections, and thus, there is a great need for alternative ways of treating thrombocytopenias.

There are numerous different types of familial thrombocytopenia, some of which show abnormal platelet surface receptors (eg, Glanzmann thrombocytopenia, Bernard-Soulier syndrome), some show abnormalities of their granule content (eg, gray platelet syndrome, storage pool platelet disease), some are associated with large platelets (benign macrothrombocytopenia), some show other syndromic features (eg, Wiskott Aldrich syndrome, May-Hegglin anomaly), and some are associated with leukemia (RUNX1 mutations). To our knowledge there is only one other published form of familial thrombocytopenia with normal sized, normally functioning platelets. This type of familial thrombocytopenia shows genetic linkage to chromosome 10p12-p11.2 (Iolascon A, et al. Pediatric Research 1999;46:548; Drachman JG, et al. Blood 2000;96:118). Many individuals with thrombocytopenia have immune-mediated thrombocytopenia (ITP). Accurate diagnosis is important so that patients receive optimal treatment, but since there is no specific test for ITP, there is a need to exclude all other forms of thrombocytopenia with specific diagnostic tests.

Buijs et al (2001) Blood 98, 2856-8 discloses a novel CBFA2 single-nucleotide mutation in familial platelet disorder with propensity to develop myeloid malignancies. Balduini et al (2002) Haematologica 87, 860-80 reviews inherited thrombocytopenias, discussing their genetic inheritance and therapy. Savoia et al (1999) Am. J. Hum. Genet. 65, 1401-5 discloses an autosomal dominant thrombocytopenia gene which maps to chromosome lOp.

### SUMMARY OF THE INVENTION

This disclosure therefore includes embodiments directed to new methods for diagnosing thrombocytopenias. Certain thrombocytopenias are genetically associated, being concentrated within a family. We have discovered a family in which a functional mutation in the protein cytochrome C, results in a condition characterized by mild thrombocytopenia, with platelet counts in the range of about 70-150 x 10⁹ platelets/L. The platelets are of normal size. The members of the family have no obvious pathophysiological symptoms, except in some cases, for a mild tendency to easy bruising and nose bleeds. Their platelet function is only mildly impaired and is described in detail below.

In particular, one mutation in cytochrome C in the affected members of the family results in the substitution of the glycine at position 42 by a serine residue. This mutation is herein termed HCS42g-s. The sequence of the DNA coding for this abnormal cytochrome C is provided, as are other oligonucleotide sequences useful for detection of HCS42g-s, the peptides and proteins containing the mutation for HCS42g-s, and antibodies useful for detecting HCS42g-s.

Accordingly the invention provides an isolated oligonucleotide having at least 14 contiguous nucleotide bases of SEQ ID NO:17 and comprising the sequence ACAAGTC or its complement.

The invention also provides an isolated peptide corresponding to a portion of the oligonucleotide of SEQ ID No: 17, comprising a region of HCS42g-s containing a sequence of 5 or more contiguous amino acids including position 42.

The invention provides an antibody capable of specifically binding to a peptide from the above protein comprising a region of HCS42g-s containing a sequence of 5 or more contiguous amino acids including position 42.

### BRIEF DESCRIPTION OF THE FIGURES

This Invention is described with respect to particular embodiments thereof. Other features and aspects of this invention are presented in the Figures, in which:
Figure 1 depicts the family tree of the pedigree with familial thrombocytopenia.
Figure 2 depicts the DNA sequence of exon 2 of cytochrome C of an affected family member.

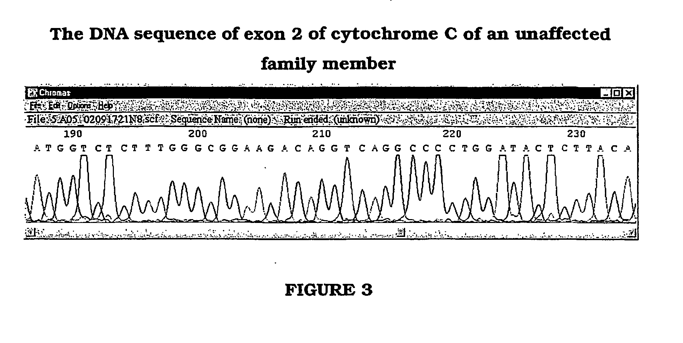

Figure 3 depicts the DNA sequence of exon 2 of cytochrome C of an unaffected family member.

### DETAILED DESCRIPTION OF THE INVENTION

In certain embodiments, this disclosure includes methods for diagnosing the presence of HCS42g-s. In certain of these embodiments, presence of HCS42g-s can be determined using nucleotide hybridization methods. In certain other embodiments, presence of HCS42g-s can be determined using antibodies directed at HCS42g-s DNA or RNA. In yet other embodiments, the HCS42g-s protein can be detected using antibodies raised against HCS42g-s or a fragment of HCS42g-s. Alternatively, specific binding by lectins can be used.

In other embodiments of this invention, HCS42g-s DNA or a fragment of HCS42g-s DNA containing the region encoding the area around position 42 can be placed in an expression vector, operably linked to a promoter, an enhancer, an initiation region, and selectable markers. In other embodiments, an expression vector containing at least a fragment of the HCS42g-s DNA can be placed into a living cell, and expression of HCS42g-s protein or a fragment thereof can be elicited. This invention also includes monoclonal and polyclonal antibodies directed towards HCS42g-s or fragments thereof containing position 42. Antibodies to HCS42g-s can be used for immunocytochemical localization of HCS42g-s, for developing detection methods including radioimmune assays (RIAs) and enzyme linked immunosorbent assays (ELISA).

In other embodiments, HCS42g-s can be detected using denaturing high pressure liquid chromatography (HPLC), by gel electrophoresis or isoelectric focusing.

In still other embodiments, HCS42g-s DNA or RNA can be detected using gel electrophoresis, HPLC methods or quantitative polymerase chain reaction (PCR).

Thus, in certain aspects, this invention includes an isolated oligonucleotide selected from SEQ ID NO: 16 or its complement, having at least 13 contiguous nucleotides including an A so that if expressed in frame, the oligonucleotide would encode a mutation at position 42 of wild-type cytochrome C.

In other aspects, this invention includes an isolated oligonucleotide selected from SEQ ID NO:16 or its complement, having at least 17 contiguous nucleotides including an A so that if expressed in frame, the oligonucleotide would encode a mutation at position 42 of wild-type cytochrome C.

In other aspects, this disclosure includes an expression vector comprising a promoter operably linked to an at least 18 mer nucleic acid having a contiguous sequence of a portion of SEQ ID NO:17 including in an open reading frame having an in-frame sequence encoding position 42 of mature HCS42g-s, or its complement.

In still further aspects, this disclosure includes a cell having an expression vector comprising a promoter operably linked to an at least 18 mer nucleic acid having a contiguous sequence of a portion of SEQ ID NO:17 including an open reading frame having an in-frame sequence encoding position 42 of mature HCS42g-s, or its complement.

Further aspects of this invention include a purified protein consisting essentially of HCS42g-s.

Still further aspects ofthis invention include an isolated peptide comprising a region of HCS42g-s containing a sequence of 5 contiguous amino acids including position 42.

Additional aspects include an antibody capable of specifically binding to a peptide comprising a region of HCS42g-s containing a sequence of 5 contiguous amino acids including position 42.

Further aspects of this invention include a device for detecting a HCS42g-s nucleic acid, comprising: a substrate; and an oligonucleotide of at least 17 mer, comprising the sequence of SEQ ID NO: 17 and including the codon encoding amino acid position 42 in said HCS42g-s, or its complement.

Additional aspects relate to a method for detecting the presence of HCS42g-s in a biological sample, comprising the steps of:
(a) providing a device, comprising:
   i a substrate; and
   ii an oligonucleotide probe of at least 17 mer, comprising the sequence of SEQ ID NO:17 and including the codon encoding amino acid position 42 in said HCS42g-s;
(b) placing onto said device, a sample containing a nucleic acid complementary to said probe; and
(c) detecting the presence of nucleic acid hybridized to said probe.

Further aspects include a method for detecting HCS42g-s protein, comprising the steps of:
(a) providing a device, comprising:
   i a substrate; and
   ii an antibody directed to at least a portion of HCS42g-s containing amino acid position 42;
(b) placing onto said device, a sample containing a HCS42g-s protein or fragment thereof containing position 42; and
(c) detecting the presence of antibody bound to said HCS42g-s protein or fragment thereof.

Additional aspects relate to a method for detecting the presence of familial thrombocytopenia in a subject, comprising: providing a sample of genetic material from said subject; and detecting the presence of one or more marker selected from the group consisting of an RFRP marker, a BE299237 marker and a Hs 72068 marker.

Yet additional aspects relate to treating familial thrombocytopenia by administering to a patient having HCS42g-s, an amount of an antisense oligonucleotide effective to decrease the expression of HCS42g-s.

Still further aspects relate to treating familial thrombocytopenia by administering to a patient having HCS42g-s, an amount of an antibody directed against HCS42g-s effective to decrease the functioning of said HCS42g-s.

### Familial Thrombocytopenia

We discovered a family having several persons with low platelet counts. Their platelet counts varied from 73 to 148 x 10⁹/L excluding one affected outlier with a count of 195 x 10⁹/L. The average platelet count of affected family members was 111 x 10⁹/L while the average count for unaffected family members was 269 x 10⁹/L (range 196 - 385). We have identified 26 related persons in whom thrombocytopenia has been documented. All share in common an ancestor, William Cargeeg, born 1831 St Just, Cornwall, England. The low platelet count is inherited in an autosomal dominant pattern. The platelets of affected family members were of normal size, but show mildly impaired function. The family members have no obvious symptoms, except perhaps a mild tendency to easy bruising and nose bleeds. Their platelet function is only mildly impaired and is described below. This is a form of thrombocytopenia that has not been identified and published previously.

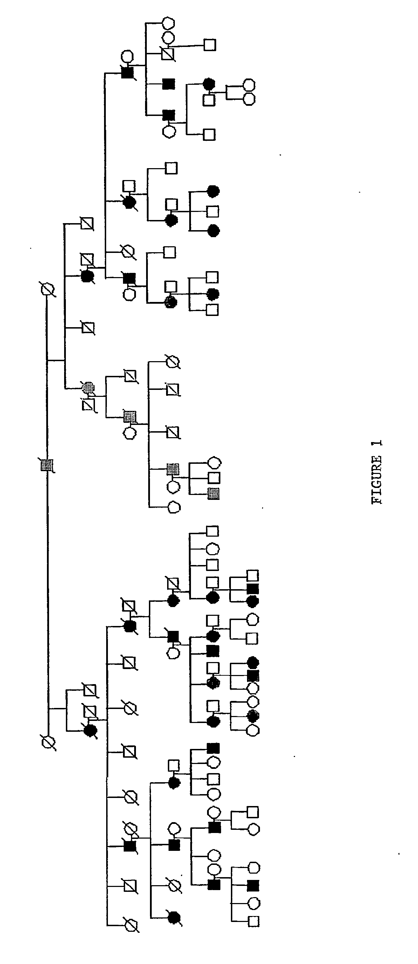

William Cargeeg was born 1831 in St. Just, Cornwall and he emigrated to New Zealand in 1875, and descendants of his two daughters (from different mothers, ie, half sisters) have inherited familial thrombocytopenia. We identified thrombocytopenia in 26 family members, and have examined DNA of 25 affected family members. The affected family members are otherwise well; we have not been able to identify any other inherited traits. Figure 1 shows the family pedigree. In Figure 1, solid symbols represent family members with thrombocytopenia. Open symbols represent family members without thrombocytopenia or whose status is unknown. Circles represent females and squares represent males.

### Platelet Function In Patients Having Cytochrome C Mutations

The platelets are of normal size as measured by the Mean Platelet Volume on a Coulter STKS hematology analyzer. They have no other recognisable syndromic features.

The platelets showed impaired aggregation in response to adrenaline and arachadonic acid, with variable response to collagen, ADP and ristocetin. Platelet nucleotide content was normal. Intra-platelet vWf:Ag levels were reduced on two occasions and low normal on one occasion.

To our knowledge there was only one other form of familial thrombcytopenia that has normal sized, normally functioning platelets. It has been described in two publications: Iolascon A, et al. Pediatric Research 1999;46:548; and Drachman JG, et al. Blood 2000;96:118. However, in these references, the genetic linkage is to chromosome 10p12-p11.2 whereas in the family we describe, the above abnormalities are attributable to HCS42g-s.

### Linkage and Sequencing

Using DNA from 25 affected family members and 38 unaffected family members and 7 spouses, we established genetic linkage of the thrombocytopenia trait to the short arm of chromosome 7. We established that all affected family members share in common a 1.12 megabase region on chromosome 7, commencing at the sequence listed below as SEQ ID NO:1.

SEQ ID NO:1 is located beginning at 24,714,661 bp on the June 2002 version of the NCBI genomic sequence of chromosome 7, and ending with the sequence:

SEQ ID NO:2 ends at 25,834,599 bp of the same sequence.

No other familial thrombocytopenic disorders were known to be linked to this region and we sequenced the DNA of selected portions of this region. We selected known genes, and expressed sequence tag sequences with significant open reading frames for sequencing.

We detected five variations in the DNA sequence that were co-inherited by all affected members of the family. One of these is a mutation in the cytochrome C gene described below. Four others are described herein. Detection of one or more of these variations or combinations of variations can be diagnostic of the familial thrombocytopenia of this invention.
1. a G/A polymorphism in the coding region of LOC136895 hypothetical protein BC015397 (unigene Hs.122055). This variation was detected by allele-specific PCR in normal persons, and in some unaffected members of the family.
2. A heterozygous homopolymer T tract addition (+/- single T) in the promoter of Rfamide (RFRP), a gene which was previously thought to be expressed only in the retina and brain. This polymorphism is not reported in the Single Nucleotide Polymorphism databases.
3. A G/A single nucleotide polymorphism in exon 2 of the EST BE299237. There are no reported polymorphisms in databases, but comparison of two EST sequences with genomic DNA sequences supports the presence of a polymorphism at this position.
4. A G/T polymorphism in unigene Hs.72068. Hs.72068 has no obvious coding capacity.

The human cytochrome C gene was sequenced using primers designed against the June 2002 release of the human genome project. This DNA sequence was downloaded from the UCSC Genome Browser website (http://genome.ucsc.edu).
The primer sequences were as follows:

| | | |
|---|---|---|
| HCSEXON1-1F | 5'-gagcgagtttggttgcactt-3' | SEQ ID NO:3 |
| HCSEXON1-1R | 5'-cagtccagggtcttcactcc-3' | SEQ ID NO:4 |
| HCSEXON2-1F | 5'-tggctagagtggtcattcattt-3' | SEQ ID NO:5 |
| HCSEXON2-1R | 5'-ctccccagatgatgcctaaa-3' | SEQ ID NO:6 |
| HCSEXON3-1-1F | 5'-acacagccgccaataagaac-3' | SEQ ID NO:7 |
| HCSEXON3-1-1R | 5'-tcaggactgcccaacaaaat-3' | SEQ ID NO:8 |
| HCSEXON3-2-1F | 5'-attttgttgggcagtcctga-3' | SEQ ID NO:9 |
| HCSEXON3-2-1R | 5'-tctgtaagatgtgagaggtgttga-3' | SEQ ID NO:10 |
| HCSEXON3-3 1F | 5'-ttggccttaactatgccaatc-3' | SEQ ID NO:11 |
| HCSEXON3-3-1R | 5'-ggctgctgcactgacattta-3' | SEQ ID NO:12 |

Oligonucleotides were purchased from Genset (http://www.gensetoligos.com/) as stocks of 100 uM.

### Methods for Analyzing Cytochrome C Mutations

The following reaction conditions were used to amplify HCS amplicons:

### Exon 1:

PCR reactions were in a 20 uL volume with the following components: 10 ng genomic DNA, 2 uL Amplitaq gold buffer, 1 mM MgCl₂, 0.5 uM each oligo (HCSEXON1-1F and HCSEXON1-1R), 125 uM dNTPs.

### Thermal cycler conditions

95 degrees C -9 min (x1)
95 degrees C - 30 sec |
56 degrees C - 30 sec |(x35)
72 degrees C - 40 sec |
72 degrees C - 6 min
12 degrees C - hold

### Exon 2:

PCR reactions were in a 20 uL volume with the following components: 10 ng genomic DNA, 2 uL Amplitaq gold buffer, 1 mM MgCl₂, 0.5 uM each oligo (HCSEXON2-1F and HCSEXON2-1R), 125 uM dNTPs.

### Thermal cycler conditions

95 degrees C -9 min (x1)
95 degrees C - 30 sec |
49 degrees C - 30 sec | (x35)
72 degrees C - 40 sec |
72 degrees C - 6 min
12 degrees C - hold

### Exon 3:

PCR reactions were in a 20 uL volume with the following components: 10 ng genomic DNA, 2 uL Amplitaq gold buffer, 1.6 mM MgCl₂, 0.5 uM each oligo (HCSEXON3-1-1F and HCSEXON3-3-1R), 125 uM dNTPs.

### Thermal cycler conditions

95 degrees C -9 min (x1)
95 degrees C - 30 sec |
54 degrees C - 30 sec |(x35)
72 degrees C - 1 min 30 sec |
72 degrees C - 6 min
12 degrees C - hold

All amplification reactions gave products having single distinct bands on 2% agarose gels. Sequences of amplification products were obtained on an ABI 3700 at the Centre for Gene Research, University of Otago using standard conditions. For HCS Exon 3, additional sequencing primers were utilised as described above and below.

### Results

The exon 1 and exon 3 sequences of each family member, including those having the famialial thrombocytopenia and those without the condition, have exon 1 and exon 3 sequences that match those of the Genbank database.

However, in patients with this familial thrombocytopenia, the exon 2 sequence is mutated. Sequences of HCS exons from affected family members are shown in SEQ ID NO:13 below. Exonic sequences are indicated in bold, and primer sequences are underlined: (note G/A heterozygote has been denoted by IUPAC code r in sequence; bold and underlined): Thus, DNA sequencing revealed a mutation at nucleotide position 132 of exon 2 of cytochrome C. The mutation results in a single base substitution: from a guanine to an adenine.

The wild type sequence of exon 2 ofhuman cytochrome C. An untranslated portion is indicated as underlined and coding triplets are separated by spaces as follows:

The coding region of human cytochrome C is as follows:

A DNA sequence of the mutated form of exon 2 from family members with thrombocytopenia follows.

The DNA sequence of the mutant protein coding region of human cytochrome C follows:

The mutation in the nucleotide sequence above results in the substitution of the glycine at position 42 by a serine residue. The glycine at position 42 is a highly conserved amino acid being invariant in 113 studied eukaryote species (Banci L et al. J Biol Inorg Chem 1999;4:824-37).

The NCBI Provisional reference sequence (NP_061820 cytochrome C [Homo sapiens]gi|11128019|ref|NP_061820.1|[11128019]) provides the following amino acid sequence for human cytochrome C. The location of the glycine that becomes mutated in HCS42g-s is indicated in bold. The sequence of the mutant cytochrome C variants in the family predicted based on the genetic code follows. The position (42) having the glycine-serine mutation is indicated in bold.

DNA from 24 family members with thrombocytopenia was then analyzed using allele-specific oligonucleotide PCR. That is, the presence of the mutation was detected with the use of the mutation-specific oligonucleotide primer (HCSexon 2+A) in combination with a reverse primer (HCSexon 2-1R). The presence of the wildtype allele was detected with the use of the wildtype-specific primer (HCSexon 2+G) together with the reverse primer (HCSexon 2-1R).
The oligonucleotide primer sequences are:

| | | |
|---|---|---|
| HCSexon 2+G | 5' gtctctttgggcggaagacag 3' | SEQ ID NO:20 |
| HCSexon 2+A | 5' gtctctttgggcggaagacaa 3' | SEQ ID NO:21 |
| HCSexon 2-1R | 5' ctccccagatgatgcctaaa 3' | SEQ ID NO:22 |

HCSexon 2+G and HCSexon 2+A are both TrueSNP LNA primers where the ultimate 3' base is a locked nucleotide. All other bases are standard nucleotides. LNA and standard primers were purchased from Proligo (www.proligo.com).

The PCR reactions were in a 20 ul volume with the following components: 10 mM Tris-HCl ph 8.3, 50 mM KCl, 4 mM MgCl2, 1 U Taq DNA polymerase (Amplitaq gold, Applied Biosystems), 125 uM each dNTP, 5 pmol each primer, 10 ng genomic DNA.

### PCR cycling conditions were:

One cycle of 95 degrees C for 9 minutes; 35 cycles of 95 degrees C for 30 seconds, 68 degrees C for 30 seconds and 72 degrees C for 30 seconds, followed by 72 degrees C for 6 minutes.

Two PCRs are performed: one with HCSexon 2+G and HCSexon 2-1R, and the other with HCSexon 2+A and HCSexon 2-1R. A third PCR utilizes any genomic DNA control primers to ensure good quality of the genomic DNA.

Using allele-specific oligonucleotide PCR the mutation was detected in 24 affected family members, whereas none of 27 non-affected family members and none of 319 non-family individuals showed the mutation. The mutation is not present in any of the RNA and EST sequences for cytochrome C in the Unigene section of GenBank, and thus represents a novel cytochrome C sequence. Unigene contains 2057 cytochrome C sequences of which approximately 950 include exon 2 sequences flanking the site of the mutation.

### Roles of Cytochrome C

Cytochrome C protein is highly conserved in all eukaryotes, with 20 internal amino acids (including glycine 42) being invariant across 113 studies species (Banci L, et al. J Biol Inorg Chem 1999;4:824-37). Cytochrome C is located in the mitochondria of all aerobic cells. It is involved in the electron transport system that functions in oxidative phosphorylation. It accepts electrons from cytochrome B and transfers them to cytochrome oxidase. In addition to its role in oxidative phosphorylation, release of cytochrome C from the mitochondrial intermembrane space results in nuclear apoptosis. Binding of APAF1 to cytochrome C allows APAF1 to form a ternary complex with, and activate, the initiator procaspase-9 in the presence of dATP. Active caspase-9 then turns on downstream effector caspases, beginning the death cascade.

Platelets are produced by large bone marrow precursor cells known as megakaryocytes. Megakaryocytes develop in the bone marrow and when they are mature their cytoplasm demarcates into long strands of cytoplasm which then fragment into platelets. Thrombopoietin is the growth factor predominantly responsible for driving megakaryocyte maturation and platelet production. The mechanisms by which platelets are produced are poorly understood, but recent evidence indicates that regional activation of apoptosis within megakaryocyte cytoplasm can play a role in platelet formation (de Botton S, et al. Blood. 2002;100(4):1310-7). Regional activation of apoptosis can involve the release of cytochrome C from mitochondria, and subsequently can result in activation of caspase 3. The role of apoptosis in platelet production is supported by the observation that platelet production is impaired by overexpression of the anti-apoptotic protein BclxL in megakaryocytes (Kaluzhny Y, et al. Blood 2002; 100(5): 1670-8).

### Diagnostic Methods for Mutated Cytochrome C

Methods for diagnosing patients having mutated cytochrome C include tests for mutant DNA, mutant RNA, and mutant protein. Suitable methods can be described in different classes. Classes of methods include cytogenetics (e.g., G-banding, FISH, CGH), hybridization (Southern blots, oligonucleotide hybridization, MutS, Northern blot, Western blot, SDS-PAGE), detection of conformation (SSCP, dideoxyfingerprinting, heteroduplex analysis, DHPCL, ethidium bromide electrophoresis, CSGE, DGGE/TGGE, cDNA gel electrophoresis, splicing abberations, paper and starch electrophoresis, and immunohistochemistry), analysis of covalent bonds (e.g., chemical mismatch cleavage, resolvases/cleavases, restriction endonucleases, sequencing, oligonucleotide ligation, RNAse cleavage, MALDI-TOF, PTT, TaqMan and ARMS). The above classes of methods and specific methods mentioned are by way of example only and are not intended to be exhaustive. These methods are summarized in Table 1 below.

**Table 1**

| **Methods for Detecting Mutations** | | | |
|---|---|---|---|
| **Class of Method** | **Starting Material** | **Technique** | **Basis of Method** |
| Cytogenetics | Living cells | G-banding | Differential staining AT rich |
| | | FISH | Hybridization |
| | | CGH | Global hybridization |
| Hybridization | DNA | Southern blot | Single-stranded probe nucleic acid |
| | | Oligonucleotide hybridization, including chips | 2-D solid-phase hybridization, 70% efficient; short probe |
| | | MutS | MutS protein detects base pair mismatches |
| | RNA | Northern blot | Alteration of nucleic acid ("n.a.") size or hybridization pattern |
| | Protein | SDS-PAGE | Detection of protein size |
| | | Western blot | Alteration in protein size; immunological detection |
| Conformation | ssDNA | SSCP | Change in denatured n.a. conformation/mobility |
| | ssDNA: denatured | Dideoxyfmger-printing | Change in denatured n.a. size, mobility or multiplicity of species |
| | dsDNA: native | Heteroduplex analysis | Change in native n.a. conformation/ mobility |
| | | DHPLC | Change in native n.a. size/mobility |
| | dsDNA: denatured | Ethidium bromide electrophoresis | Change in denatured n.a. size/mobility |
| | ss/dsDNA partially denatured | CSGE | Change in partially denatured n.a. conformation/mobility |
| | | DGGE/TGGE | Change in partially denatured n.a. conformation/mobility |
| | RNA | cDNA gel | Splicing aberrations |
| | Protein (in vivo) | Paper or starch electrophoresis | Alpha-a-AT, haemoglobins, etc. |
| | | Immuno-histochemistry | DMD, DNA, MMR proteins, etc. |
| **Changes in Covalent Bonds** | DNA cleavage | Chemical mismatch cleavage | Increased sensitivity of mismatched base pairs. |
| | | Resolvases, cleavases | Enzymes that cleave DNA secondary structure. |
| | | Restriction endonucleases | Enzymes that cleave DNA primary structure. |
| | | Sequencing | Includes minisequencing |
| | | Oligonucleotide ligation | DNA oligo ligation after hybridization. |
| | RNA | RNAse cleavage | RNA in RNA/DNA hybrid cleaved at mismatched bases by RNAse |
| | DNA/Protein | MALDI-TOF | Mass spectrometry, fragment size differences |
| | DNA | PTT | Change in size/mobility of protein generated in vitro. |
| **Mixed** | DNA/RNA | TaqMan | Differential hybridization/enzymatic destruction. |
| | | ARMS | PCR with differential oligo hybridization. |

Because the above methods as well as others are known in the art, they need not be described further herein.

### Detection of Mutant DNA and RNA

Mutant HCS42g-s DNA and RNA can be detected using methods known in the art, and including DNA sequencing, altered mobility by electrophoresis or chromatography, allele-specific amplification, oligonucleotide hybridization, and DNA cleavage. Several examples are described in Cotton RGH et al. (eds), Mutation Detection: A Practical Approach (Practical Approach Series, No 188), IRL Press, 1998, incorporated fully by reference herein.

It can be readily appreciated that the HCS42g-s DNA comprises two DNA strands, a first strand oriented in the 5'-3' direction and the other strand having a nucleotide sequence that is complementary to the first strand. It can also be readily appreciated that one can detect HCS42g-s nucleic acids by assaying a sample for the presence of either one or both strands of HCS42g-s DNA. For RNA, typically only one strand is expressed as mRNA, so it can be desirable to use a complementary probe capable of hybridizing to the mRNA of the HCS42g-s.

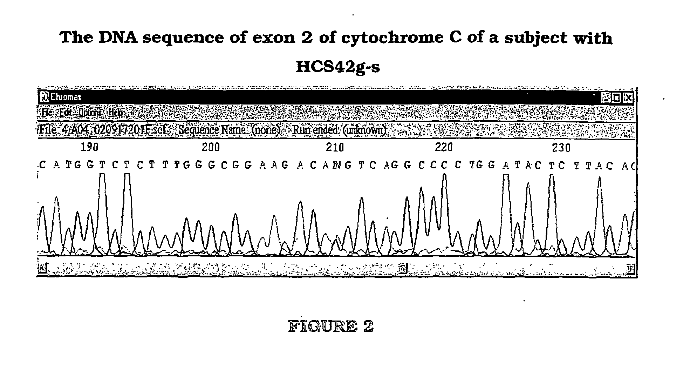

The HCS42g-s mutation and adjacent DNA or RNA sequences can be detected by DNA sequencing an example of which is shown in Figure 2. The presence of the mutant nucleotide together with approximately 20 bp of sequence on either side of the mutation is sufficient to uniquely identify the mutation.

The HCS42g-s mutation and adjacent DNA or RNA sequences can be detected by demonstration of characteristic alterations in the mobility of the mutant DNA or cDNA (complementary DNA derived from RNA) compared to wild type DNA or cDNA in electrophoretic gels or chromatography analysis systems, such as single stranded conformational polymorphism analysis, heteroduplex analysis, denaturing gradient gel electrophoresis, temperature gradient gel electrophoresis, high performance liquid chromatography, chemical cleavage analysis, and related methods. Typically in such analyses an amplified fragment of DNA or cDNA 50-500 bp in length that includes the mutation is analyzed by electrophoresis or chromatography.

HCS42g-s mutants and adjacent DNA or RNA sequences can be detected by amplifying the DNA or cDNA using an oligonucleotide primer specific for the mutant form. Specific primers usually include the mutant base at the 3' end, and have a sequence complementary to either strand of the DNA. Such primers are typically up to 40 base pairs in length.

The HCS42g-s mutation can be detected by hybridising DNA or RNA-derived cDNA to an oligonucleotide probe that is complementary to the mutant sequence. Such methods include the use of real-time quantitative PCR in which the mutant product is detected by fluorescent allele-specific probes, and solid phase assays in which the oligonucleotides complementary to the mutant sequence is attached to a surface and used to capture the mutant DNA or RNA.

Hybridization methods rely upon the difference between wild-type cytochrome C and mutant cytochrome C. Thus, suitable probes include DNA or RNA probes that are complementary to the mutant nucleotide. Probes having a continuous sequence containing the mutation (herein considered to be at position "0." Thus, probes of any length up to about 80 mer can be used. It can be appreciated that oligonucleotides having about 15 mer or greater can bind to mutant DNA having a single base substitution. Additionally, longer probes can be used if the conditions of hybridization are chosen appropriately. Moreover, convenient probe lengths include those of 17 mer to about 80 mer (-40 to +40, relative to the "0" position at amino acid 42). It can be appreciated that any oligonucleotide within the range of about -40 to about +40 and including position 0 can be used. Thus, oligonucleotides including positions -5 to +15, 0 to +20, -10 to +10, -15 to +5 and -20 to 0 are examples of 20-mer oligonucleotides that are suitable. Similarly, numerous suitable oligonucleotides can be used having any desired length, from a minimum number necessary to distinguish the mutant oligonucleotide from the wild-type or other mutant types. Thus, the minimum length in some cases can be as few as 10 mer. However, in some embodiments, longer oligonucleotides, such as 14,15 16, or 17 mer can be used. Additionally the upper limit of length of an oligonucleotide is not fixed. In some embodiments, oligonucleotides may be over 100 mer in length, so long as the probe is capable of binding sufficiently tightly to the mutant nucleic acid to be specifically detectable.

In some embodiments, sequences suitable for detecting HCS42g-s include GGG CGG AAG ACA AGT CAG GCC CCT GGA, any subset of the full-length oligonucleotide that contains the mutation. In certain embodiments, one or more subset of 13-mer oligonucleotide of the above sequence containing A from SEQ ID NO:16 in the position indicated in boldface type can be useful. Thus, for example, the following 13 mer sequences are suitable for detecting nucleic acids having the HCS42g-s mutation: AAGACAAGTCAGG, GGG CGG AAG ACA A, GG CGG AAG ACA AG, G CGG AAG ACA AGT, CGG AAG ACA AGT C, GG AAG ACA AGT CA, G AAG ACA AGT CAG, AAG ACA AGT CAG G, AG ACA AGT CAG GC, G ACA AGT CAG GCC, ACA AGT CAG GCC C, CA AGT CAG GCC CC, A AGT CAG GCC CCT, AGT CAG GCC CCT G.

It can be appreciated that longer sequences containing the nucleotide mutation of HCS42g-s that contains the nucleotide mutation encoding the protein for 42g can be used. For example, sequences from SEQ ID NO:16 like those above but having 14 mer, 15 mer, 16 mer, 17 mer, 18 mer, 19 mer, 20 mer and/or even longer sequences can be used. It can further be appreciated that oligonucleotides complementary to the above sequences can be used. Thus, a complementary oligonucleotide having 13,14,15,16,17,18,19, 20 or more nucleotides can be used satisfactorly. The only requirement is that under suitable hybridization conditions, the detection system and device can distinguish between the wild-type sequence and the mutant HCS42g-s oligonucleotide sequence.

Similarly, mutant RNA can be detected using similar strategies. Either RNA or DNA oligonucleotides can be used as probes. Moreover, chemically modified forms of oligonucleotides maybe used so long as the base pairing of A to T or U and C to G is maintained.

The HCS42g-s mutation can be detected by cleavage of DNA, RNA or cDNA by restriction enzymes, chemical mismatch cleavage, RNAse cleavage and other methods that recognize a base pair mismatch, or that identify the specific sequence of and adjacent to HCS42g-s DNA. This may involve the design of mismatched PCR primers that introduce a restriction site into the amplified product.

### Expression of HCS42g-s

Full length HCS42g-s, exon 2 of HCS42g-s, or smaller fragments of HCS42g-s can be inserted into an appropriate cell that is capable oftranslating HCS nucleic acids. Because cytochrome C is common in a wide variety of organisms, most organisms can be used to make HCS42g-s. HCS42g-s nucleic acids (DNA or RNA) can be introduced into cells either as the nucleic acid itself, or alternatively in an expression vector. Regardless of how the HCS42g-s is introduced into a cell, quantities of HCS42g-s or a portion thereof can be easily produced using methods known in the art.

In some embodiments, a selected portion of HCS42g-s is operably linked, in frame, to one or more promoter regions, optionally one or more enhancer regions, and an appropriate, optional linker so that an open reading frame includes the region of interest in frame. Additionally, an expression vector can desirably contain a selectable marker (e.g., penicillin resistance) to permit selection of cells that have the HCS42g-s nucleic acid therein. It can also be desirable to incorporate a functional enzyme into an HCS42g-s expressing cell. Such an enzyme, such as beta-galactosidase (β-Gal; including BlueScript ^{™} and others known in the art), horse radish peroxidase (HRP) and dehydrofolate reductase (DHFR) can be included in an expression vector that also has an HCS42g-s construct. Several suitable expression systems and vectors are described in Sambrook et al. Molecular Cloning, Third Edition, Cold Spring Harbor, 2001, incorporated herein fully by reference. Vectors include PBR 322 and other expression vectors are known in the art and need not be described further. It can be appreciated that one can either use a selectable marker in the same vector as the HCS42g-s or in another vector. It can also be appreciated that plasmids can be used, or alternatively, viruses can be used.

An expression vector can be transfected into an appropriate procaryotic cell (e.g., E. coli) or eukaryotic cell (e.g., 293 or COS cells or others). Numerous prokaryotic and eukaryotic cells are known in the art and need not be described herein further. Mutant HCS can also be incorporated into the genome of cells using e.g., hererologous recombination, resulting in a stably transfected cell line that can express HCS42g-s. Cells stably transfected can be selected using the selectable marker incorporated into the expression vector. Growing transfected cells *in vitro* can be accomplished using standard cell culturing conditions. Numerous methods are known for inserting vectors, plasmids or DNA into cells. Examples of such methods include calcium phosphate precipitation, electroporation, viral transfection, liposome delivery and the like. However, other methods can be used and are considered to be within the ordinary skill of a practitioner in the molecular biological arts and need not be described herein further.

In other embodiments, cell-free translation systems can be used to make HCS42g-s proteins, peptides and fragments thereof. Many of these systems are known in the art and need not be described further.

Upon sufficient cell growth, the HCS42g-s can be extracted from the cell culture medium or from the cells using known biochemical methods. It can be especially desirable to use affinity chromatography, size exclusion chromatography, gel electrophoresis, isoelectric focusing and other standard methods to isolate and purify HCS42g-s proteins or peptides.

In certain embodiments, HCS42g-s proteins or peptides can be inserted into germ line cells and then propagated *in vivo.* For example, mice can be produced that constitutively produce HCS42g-s proteins or fragments thereof. Once produced, such animals can be used to determine the biological effects of HCS42g-s. Furthermore, animals that produce HCS42g-s can be used to screen for possible therapeutic agents that can be effective in treating physiological abnormalities associated with HCS42g-s.

### Detection of Mutant Proteins

In general, HCS42g-s and its fragments can be detected using methods known in the art, including binding to monoclonal antibodies or binding to lectins or through changes in its physical structure and chemical composition. Monoclonal and polyclonal antibodies can be made against a mutant protein, which can be made using conventional techniques involving in vitro or in vivo expression systems. Thus, mutant cytochrome C protein, protein fragments, or peptides containing the mutation can be produced using recombinant methods. Once made, recombinant proteins can be purified and used as an immunogen, along with an adjuvant, if necessary, such as Freund's complete adjuvant.

Polyclonal antisera can be made using any convenient animal system, including mice and/or rabbits. Monoclonal antibodies can be raised against a mutant protein, protein fragment or mutant peptide can be made using hybridoma technologies know in the art.

Antibodies can be conveniently used for immunocytochemistry, enzyme-linked immunosorbent assays (ELISA), or radioimmunoassays (RIA). It can be appreciated that mutant proteins can be detected in any tissue that expresses the mutant cytochrome C. Thus, diagnosis of familial thrombocytopenia of this disclosure can be made from tissue biopsy specimens, from fluids obtained that contain the mutant protein, or any other source having sufficient amount of protein for analysis.

HCS42g-s can be detected using methods that analyse the sequence and composition of the protein, including protein sequencing, protein sizing methods such as two dimensional gels, chromatography, mass spectrophotometry, MALDI-TOF mass analysis, with or without pre-digestion of the protein into peptide fragments.

### Devices for Detecting Mutant Cytochrome C

Numerous devices and kits can be made for detecting mutant forms of cytochrome C DNA, RNA and proteins. Thus, in some embodiments, substrates have probes attached thereto that are complementary to the DNA or RNA for the mutant cytochrome C. Such substrates may be quartz, glass, silicon, plastic or other material to which oligonucleotide probes can be attached. Nucleotides can be attached to a substrate using any convenient linker, including sulfhydryl (SH) groups. Once prepared, a substrate may be stored in a suitable buffer, and detection of mutant nucleotide sequences made according to methods known in the art for nucleotide hybridization detection. It can be appreciated that if RNA is to be detected, it can be desirable to include in the buffer, inhibitors of RNAases. Such inhibitors are known in the art and are readily available from commercial sources. In some embodiments, sequences suitable for detecting HCS42g-s include GGG CGG AAG ACA AGT CAG GCC CCT GGA, any subset of the full-length oligonucleotide that contains the mutation. In certain embodiments, one or more subset of 13-mer oligonucleotide of the above sequence containing A from SEQ ID NO:16 in the position indicated in boldface type can be useful. Thus, for example, the following 13 mer sequences are suitable for detecting nucleic acids having the HCS42g-s mutation: AAGACAAGTCAGG, GGG CGG AAG ACA A, GG CGG AAG ACA AG, G CGG AAG ACA AGT, CGG AAG ACA AGT C, GG AAG ACA AGT CA, G AAG ACA AGT CAG, AAG ACA AGT CAG G, AG ACA AGT CAG GC, G ACA AGT CAG GCC, ACA AGT CAG GCC C, CA AGT CAG GCC CC, A AGT CAG GCC CCT, AGT CAG GCC CCT G.

It can be appreciated that devices having longer sequences (e.g., containing 14 mer, 15, 16, 17, 18., 19, 20 or more nucleotides containing the nucleotide mutation encoding the protein for HCS42g-s can be used. It can be appreciated that oligonucleotide sequences complementary to the above sequences can also be used. Moreover, to detect mutant RNAs, devices can be made that incorporate oligonucleotide probes made with U instead of T. The only requirement is that under suitable hybridization conditions, the detection system and device can distinguish between the wild-type sequence and an oligonucleotide sequence containing the mutant HCS42g-s oligonucleotide sequence.

Devices can include flat substrates such as silicon wafers, flat plastic, glass dishes or can multiwell plates. The only requirements are that the substrate be suitable for attachment of a probe (nucleotide, antibody or peptide), is suitably inexpensive, and is sufficiently inert to solutions typically used for passivating, incubation, washing and other procedures used in such analysis. Finally, the substrate should be suitably shaped to easily render detection of the analyte (nucleic acid, protein, peptide or antigen).

To assay for a nucleic acid associated with HCS42g-s, a sample of fluid or extract from cells is applied to the plate under conditions that permit hybridization of HCS42g-s nucleic acid (DNA or RNA). Buffer conditions can be selected to provide desired stringency of hybridization. Exact conditions can depend upon the amount of HCS42g-s nucleic acid present in the sample, the amount of an oligonucleotide probe bound to the test nucleic acid, the type of label on the probe, the stringency of hybridization, the sensitivity of the equipment used for detection and/or other factors. It can be desirable to design systems capable of making replicate measurements to increase accuracy, especially if quantitative determinations are desired.

Kits for detecting HCS42g-2 can include a substrate having an oligonucleotide probe, a hybridization buffer, a detection reagent capable of detecting hybridized nucleic acids, and instructions for use. Similarly, kits for quantitative PCR can contain forward and reverse PCR primers that encompass the desired region to be detected, appropriate enzymes and reagents (e.g., nucleotide triphosphates), tubes, buffers and instructions for use.

### Diagnosis of Familial Thrombocytopenia Using Markers

In addition to direct detection of HCS42g-s, embodiments of this invention also include markers for the detection of genetic polymorphisms that are associated with familial thrombocytopenia. Such markers include the following polymorphisms.
(1) A heterozygous homopolymer T tract addition (+/- single T) in the promoter of Rfamide (RFRP), a gene which was previously thought to be expressed only in the retina and brain. This polymorphism is not reported in the Single Nucleotide Polymorphism databases. Thus, this polymorphism can be characteristic of familial thrombocytopenia. This polymorphism is herein termed an "RFRP marker."
(2) A G/A single nucleotide polymorphism in exon 2 of the EST BE299237. There are no reported polymorphisms in databases, but comparison of two EST sequences with genomic DNA sequences supports the presence of a polymorphism at this position. Thus, this polymorphism can be characteristic of familial thrombocytopenia. This polymorphism is herein termed a "BE299237 marker."
(3) A G/T polymorphism in unigene Hs.72068. Hs.72068 has no obvious coding capacity. However, it can be characteristic of familial thrombocytopenia. This marker is herein termed a "Hs 72068 marker."

### Treatment of Familial Thrombocytopenia

Based on the discovery of the HCS42g-s mutant, one can decrease the expression of the mutant protein using antisense nucleotide therapies. The basis for such therapies is the use of an oligonucleotide that is complementary to the region of the strand of the DNA or RNA that encodes the mutant protein. Such antisense approaches are known in the art. In certain embodiments, it can be desirable to select an antisense sequence that hybridizes with the promoter region of a gene. In other embodiments, it can be desirable to select an antisense sequence that hybridizes to an area of the DNA or mRNA that contains the mutation. Thus, one can select one or more antisense oligonucleotides based in the sequence information contained in SEQ ID NO:14 For example the sequence AAG ACA GGT CAG GCC of SEQ ID NO: 14 contains the mutant G (indicated in bold). Thus, an antisense oligonucleotide that can hybridize with this portion of SEQ ID NO:14 includes the reverse complement: GGC CTG ACC TGT CTT. It can be appreciated that numerous other antisense oligonucleotides can be used. In certain embodiments, it is desirable to have the antisense oligonucleotide comprise more or less than the 15 mer described above. Similarly, one need not select an oligonucleotide having the mutation in the middle. As for probes suitable for detecting HCS42g-s, one can use one or more of a variety of different oligonucleotides.

To use an antisense oligonucleotide, one can either deliver the oligonucleotide as bare DNA, although this may be limited by cellular uptake and degradation of the antisense molecule. Alternatively, there are a number of derivatized antisense oligonucleotide chemistries that are known in the art, and includes phorphorothioate, phosphothionate, other sulfer-containing oligodeoxynucleotides, as well as derivatized oligoribonucleotides. Alternatively, a plasmid may be inserted into an affected cell, the plasmid containing at least a portion of a sequence that when transcribed, produces a desired antisense oligonucleotide. In further alternatives, a patients cells can be isolated and subjected to gene therapy using a plasmid containing a sequence that encodes an antisense oligonucleotide. In some of these embodiments, the plasmid can be created to have elements responsive to particular agents that can either increase or decrease the expression of the antisense-containing plasmid. Thus, after transfecting the plasmid into the cells and upon delivery of the transfected cells back into the patient, the expression of an antisense oligonucleotide therapeutic can be regulated.

It can be appreciated that in patients that are heterozygous for the HCS42g-s mutation, the use of mutation-specific antisense approaches can diminish the roles of the HCS42g-s in the patient's physiology without adversely affecting expression of the normal HCS.

As an alternative to antisense therapies, one can use antibody-based therapies to selectively bind to and inhibit adverse effects of HCS42g-s. Antibody therapies are known in the art, and given the ability of one to make antisera and monoclonal antibodies directed towards HCS42g-s, one can use those antisera or antibodies by injecting them into affected patients.

It can be appreciated that detection the above polymorphisms, either alone or in combination, can be diagnostic of familial thrombocytopenia. Especially when combined with detection of HCS42g-s, the presence of these other markers may provide greater certainty of diagnosis in conditions in which the quality of HCS42g-s measurements may be suspect.

### INDUSTRIAL APPLICABILITY

Embodiments of this invention are useful in the health care industries for diagnosis of a new type of familial thrombocytopenia, HCS42g-s. Embodiments include oligonucleotide probes capable of detecting HCS42g-s, isolated HCDS42g-2, antibodies directed against HCS42g-s, isolated nucleic acids capable of being translated into HCS42g-s or fragments thereof, cells transfected with nucleic acids encoding portions of HCS42g-s, and kits useful for detection and quantification of HCS42g-s. Additionally, methods and compositions are provided that permit detection of other mutant nucleic acids associated with HCS42g-s.

## Claims

1. An isolated oligonucleotide having at least 14 contiguous nucleotide bases of SEQ ID NO:17 and comprising the sequence ACAAGTC or its complement.

2. An isolated oligoribonucleotide having at least 14 contiguous ribonucleotide bases which are equivalent to 14 contiguous bases of SEQ ID NO:17 and comprising the sequence ACAAGUC or its complement.

3. An isolated oligonucleotide sequence of claim 1 comprising the sequence CCTGACTTGTCTT or its complement.

4. An isolated oligonucleotide of claim 1 comprising the sequence AAGACAAGTCAGG or its complement.

5. An isolated oligoribonucleotide of claim 2 comprising the sequence CCUGACUUGUCUU or its complement.

6. A isolated oligonucleotide of claim 1 comprising SEQ ID NO:17 or its complement.

7. An isolated peptide corresponding to a portion of the oligonucleotide of claim 6, comprising a region of HCS42g-s containing a sequence of 5 or more contiguous amino acids including position 42.

8. An isolated peptide of claim 7 comprising the protein having the sequence of SEQ ID NO:19.

9. A purified protein of claim 8 consisting essentially of HCS42g-s.

10. An antibody capable of specifically binding to a peptide from the protein of claim 7 comprising a region of HCS42g-s containing a sequence of 5 or more contiguous amino acids including position 42.

11. The antibody of claim 10, wherein said antibody is a monoclonal antibody.

12. An antiserum capable of specifically binding to a peptide of the protein of claim 7 comprising a region of HCS42g-s containing a sequence of 5 or more contiguous amino acids including position 42.

13. A device for detecting a HCS42g-s nucleic acid, comprising
a substrate; and
an oligonucleotide of any of claims 1-6 comprising the sequence AAGACAAGTCAGG or its complement.

14. A device of claim 13 for detecting a HCS42g-s nucleic acid, comprising:
a substrate; and
an oligonucleotide of at least 17 mer from the sequence of SEQ ID NO: 17, said oligonucleotide including the codon encoding amino acid position 42 in said HCS42g-s.

15. A device for detecting a HCS42g-s nucleic acid, comprising
a substrate; and
an oligonucleotide selected from the group consisting of AAGACAAGTCAGG, GGG CGG AAG ACA A, GG CGG AAG ACA AG, G CGG AAG ACA AGT, CGG AAG ACA AGT C, GG AAG ACA AGT CA, G AAG ACA AGT CAG, AAG ACA AGT CAG G, AG ACA AGT CAG GC, G ACA AGT CAG GCC, ACA AGT CAG GCC C, CA AGT CAG GCC CC, A AGT CAG GCC CCT and AGT CAG GCC CCT G or complements thereof.

## Patentansprüche

1. Isoliertes Oligonukleotid, das wenigstens 14 aufeinander folgende Nukleotidbasen der SEQ ID NR. 17 aufweist und das die Sequenz ACAAGTC oder deren Komplement umfasst.

2. Isoliertes Oligoribonukleotid, das wenigstens 14 aufeinander folgende Ribonukleotidbasen aufweist, die äquivalent zu den 14 aufeinander folgende Basen der SEQ ID NR. 17 sind, und das die Sequenz ACAAGUC oder deren Komplement umfasst.

3. Isolierte Oligonukleotidsequenz nach Anspruch 1, umfassend die Sequenz CCTGACTTGTCTT oder deren Komplement.

4. Isoliertes Oligonukleotid nach Anspruch 1, umfassend die Sequenz AAGACAAGTCAGG oder deren Komplement.

5. Isoliertes Oligoribonukleotid nach Anspruch 2, umfassend die Sequenz CCUGACUUGUCUU oder deren Komplement.

6. Isoliertes Oligonukleotid nach Anspruch 1, umfassend die SEQ ID NR. 17 oder deren Komplement.

7. Isoliertes Peptid entsprechend einem Abschnitt des Oligonukleotids nach Anspruch 6, umfassend eine Region von HCS42g-2, die eine Sequenz von 5 oder mehr aufeinander folgenden Aminosäuren einschließlich der Position 42 enthält.

8. Isoliertes Peptid nach Anspruch 7, umfassend das Protein mit der Sequenz der SEQ ID NR. 19.

9. Gereinigtes Protein nach Anspruch 8, im Wesentlichen bestehend aus HCS42g-s.

10. Antikörper, der zur spezifischen Bindung an ein Peptid aus dem Protein nach Anspruch 7 fähig ist, umfassend eine Region von HCS42g-s, die eine Sequenz von 5 oder mehr aufeinander folgenden Aminosäuren einschließlich der Position 42 enthält.

11. Antikörper nach Anspruch 10, wobei der Antikörper ein monoklonaler Antikörper ist.

12. Antiserum, das zur spezifischen Bindung an ein Peptid aus dem Protein nach Anspruch 7 fähig ist, umfassend eine Region von HCS42g-s, die eine Sequenz von 5 oder mehr aufeinander folgenden Aminosäuren einschließlich der Position 42 enthält.

13. Element zum Nachweisen einer HCS42g-s-Nukleinsäure, umfassend:
ein Substrat; und
ein Oligonukleotid nach einem der Ansprüche 1-6, umfassend die Sequenz AAGACAAGTCAGG oder deren Komplement.

14. Element nach Anspruch 13 zum Nachweisen einer HCS42g-s-Nukleinsäure, umfassend:
ein Substrat; und
ein Oligonukleotid von wenigstens einem 17-mer aus der Sequenz der SEQ ID NR. 17, welches Oligonukleotid das Codon enthält, das die Aminosäure-Position 42 in der HCS42g-s codiert..

15. Element zum Nachweisen einer HCS42g-s-Nukleinsäure, umfassend:
ein Substrat; und
ein Oligonukleotid, ausgewählt aus der Gruppe, bestehend aus AAGACAAGTCAGG, GGG CGG AAG ACA A, GG CGG AAG ACA AG, G CGG AAG ACA AGT, CGG AAG ACA AGT C, GG AAG ACA AGT CA, G AAG ACA AGT CAG, AAG ACA AGT CAG G, AG ACA AGT CAG GC, G ACA AGT CAG GCC, ACA AGT CAG GCC C, CA AGT CAG GCC CC, A AGT CAG GCC CCT und AGT CAG GCC CCT G oder Komplemente davon.

## Revendications

1. Oligonucléotide isolé ayant au moins 14 bases nucléotidiques contiguës de la SEQ ID N° 17 et comprenant la séquence ACAAGTC ou son complément.

2. Oligoribonucléotide isolé ayant au moins 14 bases ribonucléotidiques contiguës qui sont équivalentes à 14 bases contiguës de la SEQ ID N° 17 et comprenant la séquence ACAAGUC ou son complément.

3. Séquence d'oligonucléotide isolé selon la revendication 1 comprenant la séquence CCTGACTTGTCTT ou son complément.

4. Oligonucléotide isolé selon la revendication 1 comprenant la séquence AAGACAAGTCAGG ou son complément.

5. Oligoribonucléotide isolé selon la revendication 2 comprenant la séquence CCUGACUUGUCUU ou son complément.

6. Oligonucléotide isolé selon la revendication 1 comprenant la SEQ ID N° 17 ou son complément.

7. Peptide isolé correspondant à une portion de l'oligonucléotide selon la revendication 6, comprenant une région de HCS42g-s contenant une séquence de 5 aminoacides contigus ou plus incluant la position 42.

8. Peptide isolé selon la revendication 7 comprenant la protéine ayant la séquence de SEQ ID N° 19.

9. Protéine purifiée selon la revendication 8 consistant essentiellement en HCS42g-s.

10. Anticorps capable de se lier spécifiquement à un peptide de la protéine selon la revendication 7 comprenant une région de HCS42g-s contenant une séquence de 5 aminoacides contigus ou plus incluant la position 42.

11. Anticorps selon la revendication 10, dans lequel ledit anticorps est un anticorps monoclonal.

12. Antisérum capable de se lier spécifiquement à un peptide d'une protéine selon la revendication 7 comprenant une région HCS42g-s contenant une séquence de 5 aminoacides contigus ou plus incluant la position 42.

13. Dispositif pour détecter un acide nucléique HCS42g-s, comprenant
un substrat, et
un oligonucléotide selon l'une quelconque des revendications 1 à 6 comprenant la séquence AAGACAAGTCAGG ou son complément.

14. Dispositif selon la revendication 13 pour détecter un acide nucléique HCS42g-s, comprenant
un substrat, et
un oligonucléotide d'au moins 17-mer de la séquence de SEQ ID N° 17, ledit oligonucléotide incluant le codon codant pour un acide aminé en position 42 dans ledit HCS42g-s.

15. Dispositif pour détecter un acide nucléique HCS42g-s, comprenant
un substrat, et
un oligonucléotide choisi parmi le groupe consistant en AAGACAAGTCAGG, GGG CGG AAG ACA A, GG CGG AAG ACA AG, G CGG AAG ACA AGT, CGG AAG ACA AGT C, GG AAG ACA AGT CA, G AAG ACA AGT CAG, AAG ACA AGT CAG G, AG ACA AGT CAG GC, G ACA AGT CAG GCC, ACA AGT CAG GCC C, CA AGT CAG GCC CC, A AGT CAG GCC CCT et AGT CAG GCC CCT G ou des compléments de ceux-ci.


### SEQUENCE LISTING

<110> Pacific Edge Biotechnology Limited Morison, Ian M
<120> VARIANTS OF CYTOCHROME C AND METHODS OF DIAGNOSIS OF THROMBOCYTOPENIA
<130> PEBL-1004WO0 DBB
<150> 60/446,416
   <151> 2003-02-11
<160> 22
<170> PatentIn version 3.2
<210> 1
   <211> 67
   <212> DNA
   <213> Homo sapians
<400> 1
   atctctatta aacacacaca cacacacaca cacacacaca cacacacaca cacacacaaa 60
   tcaactc 67
<210> 2
   <211> 52
   <212> DNA
   <213> Homo Sapians
<400> 2
   gcgccgggac ccgcgggcgc cggcaggggc gttcccgggc gcgcggcggc ga 52
<210> 3
   <211> 20
   <212> DNA
   <213> Homo Sapians
<400> 3
   gagcgagttt ggttgcactt 20
<210> 4
   <211> 20
   <212> DNA
   <213> Homo sapians
<400> 4
   cagtccaggg tcttcactcc 20
<210> 5
   <211> 22
   <212> DNA
   <213> Homo Sapians
<400> 5
   tggctagagt ggtcattcat tt 22
<210> 6
   <211> 20
   <212> DNA
   <213> Homo Sapians
<400> 6
   ctccccagat gatgcctaaa 20
<210> 7
   <211> 20
   <212> DNA
   <213> Homo sapians
<400> 7
   acacagccgc caataagaac 20
<210> 8
   <211> 20
   <212> DNA
   <213> Homo sapians
<400> 8
   tcaggactgc ccaacaaaat 20
<210> 9
   <211> 20
   <212> DNA
   <213> Homo sapians
<400> 9
   attttgttgg gcagtcctga 20
<210> 10
   <211> 24
   <212> DNA
   <213> Homo sapians
<400> 10
   tctgtaagat gtgagaggtg ttga 24
<210> 11
   <211> 21
   <212> DNA
   <213> Homo Sapians
<400> 11
   ttggccttaa ctatgccaat c 21
<210> 12
   <211> 20
   <212> DNA
   <213> Homo Sapians
<400> 12
   ggctgctgca ctgacattta 20
<210> 13
   <211> 370
   <212> DNA
   <213> Homo Sapians
<400> 13
   tggctagagt ggtcattcat ttacactgta tttgttacct gaggaaaaat ttactaagtt 60
   gaagctttcg tttttagaat taaatatggg tgatgttgag aaaggcaaga agatttttat 120
   tatgaagtgt tcccagtgcc acaccgttga aaagggaggc aagcacaaga ctgggccaaa 180
   tctccatggt ctctttgggc ggaagacarg tcaggcccct ggatactctt acacagccgc 240
   caataagaac aaaggtaaga gtcacttgtt aaataaaaca acacaaaatg caggaatata 300
   acatgtggca aactatcagg agtgtgaaat aaccgatgca ttctttcttg tttaggcatc 360
   atctggggag 370
<210> 14
   <211> 177
   <212> DNA
   <213> Homo sapians
<400> 14
   aattaaatat gggtgatgtt gagaaaggca agaagatttt tattatgaag tgttcccagt 60
   gccacaccgt tgaaaaggga ggcaagcaca agactgggcc aaatctccat ggtctctttg 120
   ggcggaagac aggtcaggcc cctggatact cttacacagc cgccaataag aacaaag 177
<210> 15
   <211> 318
   <212> DNA
   <213> Homo Sapians
<400> 15
   atgggtgatg ttgagaaagg caagaagatt tttattatga agtgttccca gtgccacacc 60
   gttgaaaagg gaggcaagca caagactggg ccaaatctcc atggtctctt tgggcggaag 120
   acaggtcagg cccctggata ctcttacaca gccgccaata agaacaaagg catcatctgg 180
   ggagaggata cactgatgga gtatttggag aatcccaaga agtacatccc tggaacaaaa 240
   atgatctttg tcggcattaa gaagaaggaa gaaagggcag acttaatagc ttatctcaaa 300
   aaagctacta atgagtaa 318
<210> 16
   <211> 177
   <212> DNA
   <213> Homo Sapians
<400> 16
   aattaaatat gggtgatgtt gagaaaggca agaagatttt tattatgaag tgttcccagt 60
   gccacaccgt tgaaaaggga ggcaagcaca agactgggcc aaatctccat ggtctctttg 120
   ggcggaagac aagtcaggcc cctggatact cttacacagc cgccaataag aacaaag 177
<210> 17
   <211> 318
   <212> DNA
   <213> Homo Sapians
<400> 17
   atgggtgatg ttgagaaagg caagaagatt tttattatga agtgttccca gtgccacacc 60
   gttgaaaagg gaggcaagca caagactggg ccaaatctcc atggtctctt tgggcggaag 120
   acaagtcagg cccctggata ctcttacaca gccgccaata agaacaaagg catcatctgg 180
   ggagaggata cactgatgga gtatttggag aatcccaaga agtacatccc tggaacaaaa 240
   atgatctttg tcggcattaa gaagaaggaa gaaagggcag acttaatagc ttatctcaaa 300
   aaagctacta atgagtaa 318
<210> 18
   <211> 105
   <212> PRT
   <213> Homo Sapians
<400> 18
   Met Gly Asp Val Glu Lys Gly Lys Lys Ile Phe Ile Met Lys Cys Ser
   1 5 10 15
   Gln Cys His Thr Val Glu Lys Gly Gly Lys His Lys Thr Gly Pro Asn
   20 25 30
   Leu His Gly Leu Phe Gly Arg Lys Thr Gly Gln Ala Pro Gly Tyr Ser
   35 40 45
   Tyr Thr Ala Ala Asn Lys Asn Lys Gly Ile Ile Trp Gly Glu Asp Thr
   50 55 60
   Leu Met Glu Tyr Leu Glu Asn Pro Lys Lys Tyr Ile Pro Gly Thr Lys
   65 70 75 80
   Met Ile Phe Val Gly Ile Lys Lys Lys Glu Glu Arg Ala Asp Leu Ile
   85 90 95
   Ala Tyr Leu Lys Lys Ala Thr Asn Glu
   100 105
<210> 19
   <211> 105
   <212> PRT
   <213> Homo Sapians
<400> 19
   Met Gly Asp Val Glu Lys Gly Lys Lys Ile Phe Ile Met Lys Cys Ser
   1 5 10 15
   Gln Cys His Thr Val Glu Lys Gly Gly Lys His Lys Thr Gly Pro Asn
   20 25 30
   Leu His Gly Leu Phe Gly Arg Lys Thr Ser Gln Ala Pro Gly Tyr Ser
   35 40 45
   Tyr Thr Ala Ala Asn Lys Asn Lys Gly Ile Ile Trp Gly Glu Asp Thr
   50 55 60
   Leu Met Glu Tyr Leu Glu Asn Pro Lys Lys Tyr Ile Pro Gly Thr Lys
   65 70 75 80
   Met Ile Phe Val Gly Ile Lys Lys Lys Glu Glu Arg Ala Asp Leu Ile
   85 90 95
   Ala Tyr Leu Lys Lys Ala Thr Asn Glu
   100 105
<210> 20
   <211> 21
   <212> DNA
   <213> Homo Sapians
<400> 20
   gtctctttgg gcggaagaca g 21
<210> 21
   <211> 21
   <212> DNA
   <213> Homo Sapians
<400> 21
   gtctctttgg gcggaagaca a 21
<210> 22
   <211> 20
   <212> DNA
   <213> Homo Sapians
<400> 22
   ctccccagat gatgcctaaa 20